# FASCICULE DE BREVET EUROPEEN

(11) **EP 2 487 158 B1**
(45) Date de publication et mention de la délivrance du brevet: **20.02.2013**
(21) Numéro de dépôt: 12290050.9
(22) Date de dépôt: 13.02.2012
(51) Int. Cl.: C07D 223/16, C07C 211/00

(54) **Nouveau procédé de synthèse de l'ivabradine et de ses sels d'addition à un acide pharmaceutiquement acceptable**
Neues Syntheseverfahren von Ivabradin und seinen Salzen als Zusatz zu einer pharmazeutisch akzeptierbaren Säure
New method for synthesising ivabradine and its added salts with a pharmaceutically acceptable acid.

(30) Priorité: 14.02.2011 FR 1100446
(43) Date de publication de la demande: 15.08.2012
(73) Titulaire: Les Laboratoires Servier, 92284 Suresnes Cedex (FR)
(72) Inventeur: Peglion, Jean-Louis, 78110 Le Vesinet (FR); Dessinges, Aimée, 92500 Rueil-Malmaison (FR)

(56) Documents cités:
- EP-A1- 2 202 225
- EP-A1- 2 241 553
- WO-A1-2005/110993
- WO-A1-2010/072409

## Description

La présente invention concerne un procédé de synthèse de l'ivabradine de formule (I) : 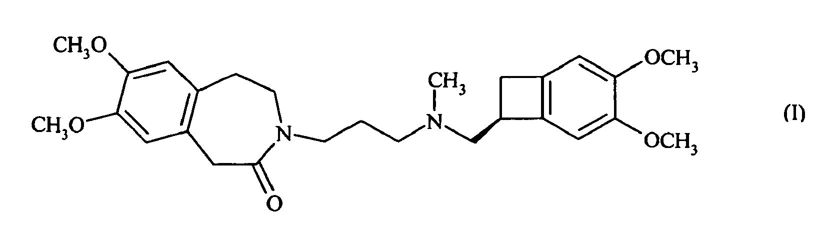 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859.

Ce brevet décrit la synthèse du chlorhydrate de l'ivabradine à partir du composé de formule (II) : 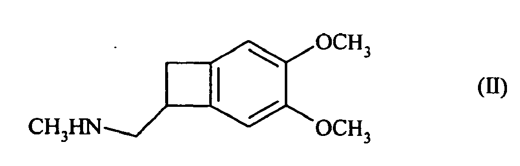 qui est dédoublé pour conduire au composé de formule (III) : 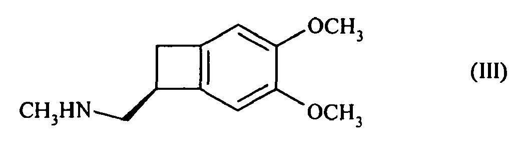 qui est mis en réaction avec le composé de formule (IV) : 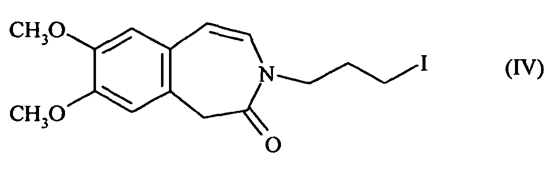 pour conduire au composé de formule (V) : 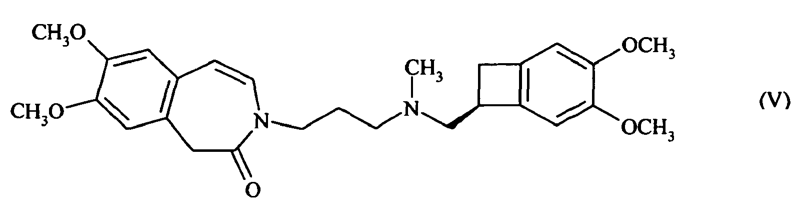 dont l'hydrogénation catalytique conduit à l'ivabradine, qui est alors transformée en son chlorhydrate.

L'inconvénient de cette voie de synthèse est de ne conduire à l'ivabradine qu'avec un rendement de 1 %.

Compte-tenu de l'intérêt pharmaceutique de ce composé, il était important de pouvoir y accéder avec un procédé de synthèse performant, conduisant à l'ivabradine avec un bon rendement.

La présente invention concerne un procédé de synthèse de l'ivabradine de formule (I): 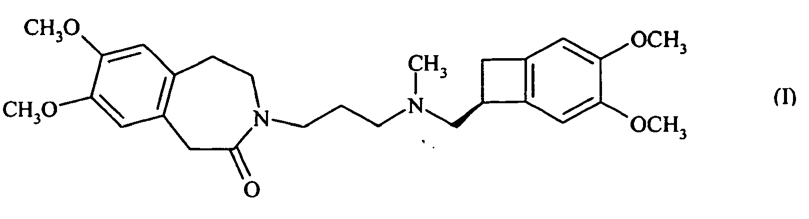 **caractérisé en ce que** le composé de formule (VI): 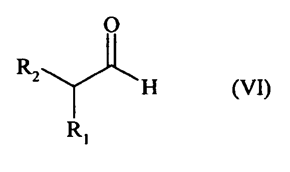 dans laquelle R₁ et R₂, identiques ou différents, représentent des groupements alkoxy (C₁-C₆) linéaires ou ramifiés, ou bien forment ensemble avec l'atome de carbone qui les porte un cycle 1,3-dioxane, 1,3-dioxolane ou 1,3-dioxépane,
est soumis à une réaction d'amination réductrice par le composé de formule (VII) : 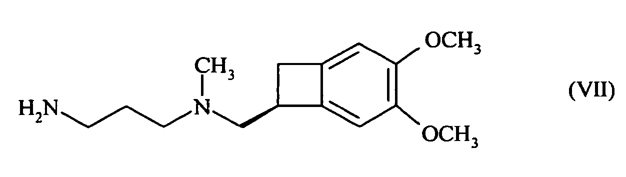 en présence d'un agent de réduction,
dans un solvant organique, un mélange de solvants organiques ou un mélange de solvant(s) organique(s) et d'eau,
pour conduire au composé de formule (VIII) : 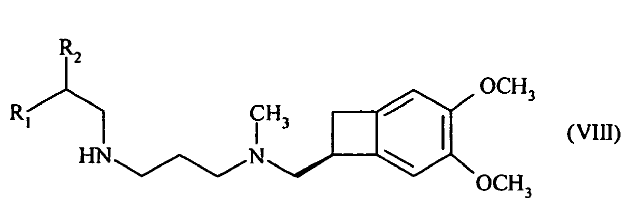 dans laquelle R₁ et R₂, sont tels que définis précédemment,
lequel est soumis à une réaction de condensation avec le composé de formule (IX) : 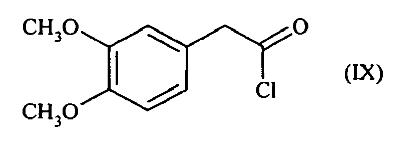 en présence d'une base dans un solvant organique,
pour conduire au composé de formule (X) : 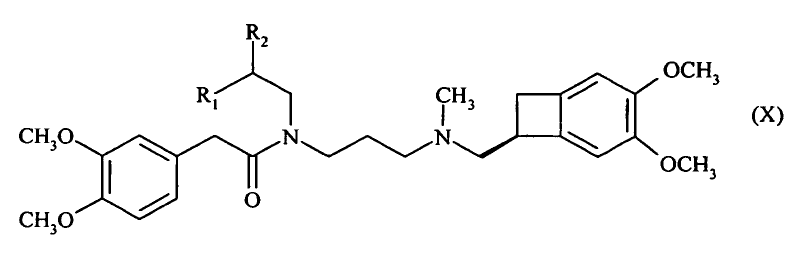 dans laquelle R₁ et R₂, sont tels que définis précédemment,
lequel est soumis à une réaction de cyclisation en milieu acide pour conduire au composé de formule (V) : 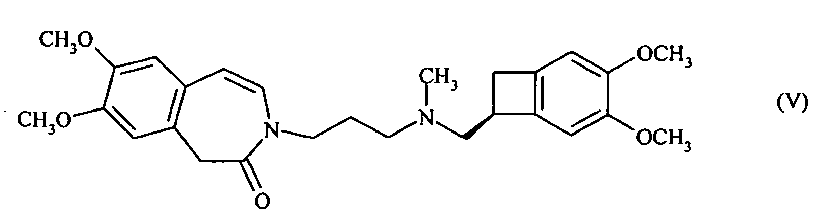 lequel est soumis à une réaction d'hydrogénation pour conduire à l'ivabradine de formule (I), qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Une liste d'agents de réduction pouvant être utilisés pour effectuer une réaction d'amination réductrice est disponible dans les ouvrage de référence Comprehensive Organic Transformations (Richard C. Larock, VCH Publishers 1989, pp 421-425) et Advanced Organic Chemistry Fourth Edition (Jerry March, Wiley Interscience 1992, pp 898-900).

Parmi les agents de réduction pouvant être utilisés pour effectuer la réaction d'amination réductrice du composé de formule (VI) par le composé de formule (VII), on peut citer à titre non limitatif le triacétoxyborohydrure de sodium, le cyanoborohydrure de sodium, et le dihydrogène en présence d'un catalyseur tel que le palladium, le platine, le nickel, le ruthénium, le rhodium, ainsi que leurs dérivés, notamment sous forme supportée ou sous forme d'oxydes.

L'agent de réduction préférentiellement utilisé pour effectuer la réaction d'amination réductrice du composé de formule (VI) par le composé de formule (VII) est le dihydrogène en présence de palladium sur charbon.

La réaction d'amination réductrice du composé de formule (VI) par le composé de formule (VII) est préférentiellement conduite à une pression de dihydrogène comprise entre 0,5 et 1,5 bar.

Parmi les solvants pouvant être utilisés pour effectuer la réaction d'amination réductrice du composé de formule (VI) par le composé de formule (VII), on peut citer à titre non limitatif le tétrahydrofurane, le dichlorométhane, le 1,2-dichloroéthane, les acétates, les alcools, préférentiellement l'éthanol, le méthanol ou l'isopropanol, le toluène et le xylène.

Le solvant préférentiellement utilisé pour effectuer la réaction d'amination réductrice du composé de formule (VI) par le composé de formule (VII) est constitué par un mélange d'éthanol et d'eau.

La réaction d'amination réductrice du composé de formule (VI) par le composé de formule (VII) est préférentiellement conduite à une température comprise entre 0°C et 40°C.

Parmi les solvants organiques pouvant être utilisés dans la réaction entre les composés de formule (VIII) et (IX), on peut citer à titre non limitatif le toluène, le dichlorométhane, le 2-méthyltétrahydrofurane, le chlorobenzène, le 1,2-dichloroéthane, le chloroforme et le dioxane.

Le solvant organique préférentiellement utilisé dans la réaction entre les composés de formule (VIII) et (IX) est le dichlorométhane.

La réaction entre les composés de formule (VIII) et (IX) est préférentiellement conduite à une température comprise entre 0 et 40°C.

Parmi les bases qui peuvent être utilisées dans la réaction entre les composés de formule (VIII) et (IX), on peut citer à titre non limitatif la pyridine, la DMAP et les amines tertiaires, par exemple la triéthylamine, la DIEA, la *N*-méthylpipéridine, la DBU, le DABCO, le DBN et la *N*-méthylmorpholine.

La base préférentiellement utilisée dans la réaction entre les composés de formule (VIII) et (IX) est la triéthylamine.

Parmi les acides pouvant être utilisés pour effectuer la cyclisation du composé de formule (X) en composé de formule (V), on peut citer à titre non limitatif l'acide sulfurique concentré, l'acide polyphosphorique, l'acide chlorhydrique concentré en solution aqueuse, l'acide chlorhydrique concentré en solution dans l'acide acétique, l'acide bromhydrique concentré en solution dans l'acide acétique et l'acide méthanesulfonique.

L'acide préférentiellement utilisé pour effectuer la cyclisation du composé de formule (X) en composé de formule (V) est l'acide chlorhydrique concentré en solution dans l'acide acétique.

La réaction de cyclisation en milieu acide du composé de formule (X) en composé de formule (V) est préférentiellement conduite à une température comprise entre 0 et 40°C.

Les composés de formule (VIII) et (X) sont des produits nouveaux, utiles comme intermédiaires de synthèse dans l'industrie chimique ou pharmaceutique, notamment dans la synthèse de l'ivabradine, de ses sels d'addition à un acide pharmaceutiquement acceptable et de leurs hydrates, et font à ce titre partie intégrante de la présente invention.

### Liste des abréviations utilisées :

DABCO : 1,4-diazabicyclo[2.2.2]octane
DBN : 1,5-diazabicyclo[4.3.0]non-5-ène
DBU : 1,8-diazabicyclo[5.4.0]undec-7-ène
DIEA : *N*,*N*-diisopropyléthylamine
DMAP : 4-diméthylaminopyridine
IR : infrarouge

Les exemples ci-dessous illustrent l'invention.

Les spectres infrarouge ont été enregistrés sur un appareil Infra-rouge Bruker, tensor 27, accessoire ATR Golden Gate. Les produits sont déposés purs sur la platine.

### EXEMPLE 1 : 2-{3-[{[(7S)-3,4-diméthoxybicyclo[4.2.0]octa-1,3,5-trién-7-yl]méthyl} (méthyl)amino]propyl}-1H-isoindole-1,3(2H)-dione

On solubilise, dans 230 mL d'acétone, 5,3g (25,5 mmoles) de 1-[(7*S*)-3,4-diméthoxybicyclo[4.2.0]octa-1,3,5-trièn-7-yl]-*N*-méthylméthanamine et 6,8g (25,5 mmoles) de 2-(3-bromopropyl)-1*H*-isoindole-1,3(2*H*)-dione. On ajoute à cette solution 13g (95 mmoles , 3,7 éq.) de carbonate de potassium. Le mélange est alors chauffé 24 heures à reflux. Après retour à l'ambiante, le carbonate de potassium est filtré et le filtrat est évaporé à sec. Le résidu est repris à l'eau et extrait au dichlorométhane. La phase organique est séchée sur MgSO₄, filtrée et évaporée à sec. On obtient 9,7g de produit attendu sous la forme d'une huile jaune pâle.

Rendement = 97%
IR : ν = 2782, 1770, 1704, 1206, 836, 718 cm⁻¹.

### EXEMPLE 2 : N-{[(7S)-3,4-diméthoxybicyclo[4.2.0]octa-1,3,5-trién-7-yl]méthyl}-N-méthylpropane-1,3-diamine

On solubilise 9,7g (24,58 mmoles) de dérivé phtalimide de l'étape précédente dans 100 mL d'éthanol. On ajoute 2,7 mL (36,87 mmoles , 1,5 éq.) d'hydrate d'hydrazine et on chauffe à reflux pendant 4 heures. Après retour à l'ambiante, 100 mL d'une solution aqueuse d'acide chlorhydrique (4N) sont ajoutés, le mélange est agité 1 heure à température ambiante et filtré sur fritté. Le filtrat est alors évaporé (élimination de l'éthanol). Puis la phase aqueuse est lavée 2 fois à l'éther, amenée pH = 9 par ajout à froid d'une solution d'hydroxyde de sodium concentrée. On extrait 3 fois au dichlorométhane, puis les phases organiques combinées sont lavées à l'eau, séchée sur MgSO₄, filtrées et évaporées à sec. On obtient 4,9g de produit attendu sous la forme d'une huile jaune pâle.

Rendement = 75%
IR : ν = 3366, 3302, 1591 cm⁻¹.

### EXEMPLE 3 : N-{[(7S)-3,4-diméthoxybicyclo[4.2.0]octa-1,3,5-trién-7-yl]méthyl}-N'-(2,2-diméthoxyethyl)-N-méthylpropane-1,3-diamine

On met en solution dans 20 mL d'éthanol 1g (3,7 mmoles) de *N*-{[(7*S*)-3,4-diméthoxybicycio[4.2.0]octa-1,3,5-trièn-7-yl]méthyl}-*N*-méthylpropane-1,3-diamine. On ajoute 520 mg (0,45 mL) d'une solution de glyoxal-1,1-diméthyl acétal à 60% dans l'eau puis 100 mg de Pd/C à 10%. Le milieu réactionnel est hydrogéné à pression atmosphérique et température ambiante pendant 12 heures. On filtre le catalyseur et on évapore à sec le filtrat. On obtient 1,2 g de produit attendu sous la forme d'une huile.

Rendement = 90%
IR : ν = 1207, 1508, 834 cm⁻¹

### EXEMPLE 4 : N-{3-[{[(7S)-3,4-diméthoxybicyclo[4.2.0]octa-1,3,5-trién-7-yl]méthyl}(méthyl)amino]propyl}-N-(2,2-diméthoxyethyl)-2-(3,4-diméthoxyphenyl)acétamide

On prépare une solution de 6,3 g (17,9 mmoles) d'acétal de l'étape précédente dans 80 mL de CH₂Cl₂. On ajoute 5 mL de triéthylamine (35,8 mmoles , 2 éq.) à cette solution qui est alors refroidie à 0°C. On y ajoute, goutte à goutte, une solution de 3,8 g (17,9 mmoles) de chlorure d'homovératryle dans 40 mL de dichlorométhane. On agite ensuite 3 heures à température ambiante. Le milieu est dilué à l'eau et extrait au dichlorométhane. La phase organique est séchée sur MgSO₄, filtrée et évaporée à sec. On obtient 10g d'une huile qui est purifiée sur 500g de gel de silice (éluant = CH₂Cl₂ / EtOH : 90 / 10). On obtient 8,5 g de produit attendu sous forme d'une huile marron.

Rendement = 90%
IR : ν = 1627, 1207, 1124, 1071, 1049, 1027 cm⁻¹.

### EXEMPLE 5 : 3-{3-[{[(7S)-3,4-diméthoxybicyclo[4.2.0]octa-1,3,5-trién-7-yl]méthyl}(méthyl)amino]propyl}-7,8-diméthoxy-1,3-dihydro-2H-3-benzazépin-2-one

A un mélange de 10 mL d'acide acétique et 10 mL d' acide chlorhydrique concentré on ajoute 1 g (1,9 mmole) d'acétal de l'étape précédente à température ambiante. On agite 1 heure à 25°C. La solution est amenée à pH = 9 par addition de glace et d'une solution aqueuse d'hydroxyde de sodium (20%). Le milieu est ensuite extrait au dichlorométhane. La phase organique est lavée à l'eau, séchée sur MgSO₄, filtrée et évaporée à sec. On obtient 1 g d'une huile qui est purifiée par flash-chromatographie sur 40g de silice (colonne Merck™, éluant = CH₂Cl₂ / EtOH : 95 / 5). On obtient 270 mg de produit attendu sous la forme d'une huile de pureté optique supérieure à 99%.

Rendement =31%
IR : ν = 1656, 836, 760 cm⁻¹.

### EXEMPLE 6 : 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

Le composé du titre est obtenu en reproduisant le stade D de l'exemple 1 du brevet EP 0 534 859 à partir du composé de l'Exemple 5.

## Revendications

1. Procédé de synthèse de l'ivabradine de formule (I): 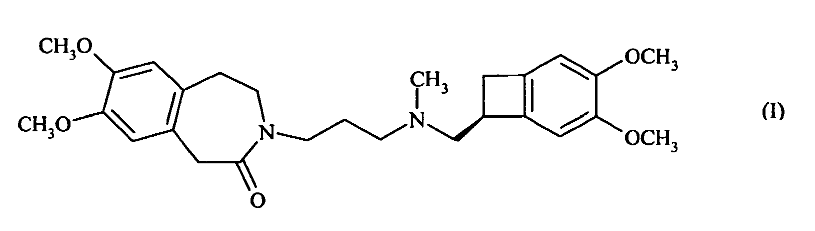 **caractérisé en ce que** le composé de formule (VI): 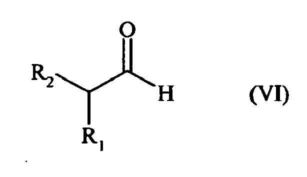 dans laquelle R₁ et R₂, identiques ou différents, représentent des groupements alkoxy (C₁-C₆) linéaires ou ramifiés, ou bien forment ensemble avec l'atome de carbone qui les porte un cycle 1,3-dioxane, 1,3-dioxolane ou 1,3-dioxépane,
est soumis à une réaction d'amination réductrice par le composé de formule (VII) : 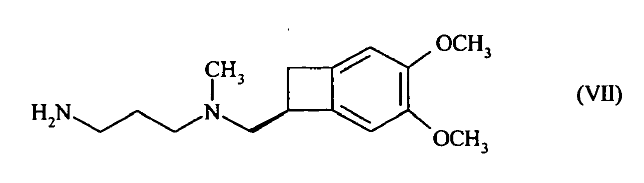 en présence d'un agent de réduction,
dans un solvant organique, un mélange de solvants organiques ou un mélange de solvant(s) organique(s) et d'eau,
pour conduire au composé de formule (VIII) : 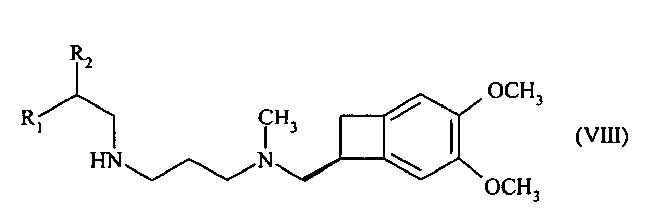 dans laquelle R₁ et R₂, sont tels que définis précédemment,
lequel est soumis à une réaction de condensation avec le composé de formule (IX) : 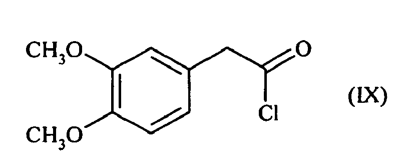 en présence d'une base dans un solvant organique, pour conduire au composé de formule (X) : 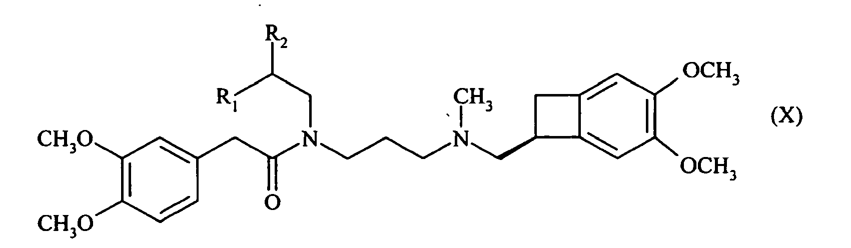 dans laquelle R₁ et R₂, sont tels que définis précédemment,
lequel est soumis à une réaction de cyclisation en milieu acide pour conduire au composé de formule (V) : 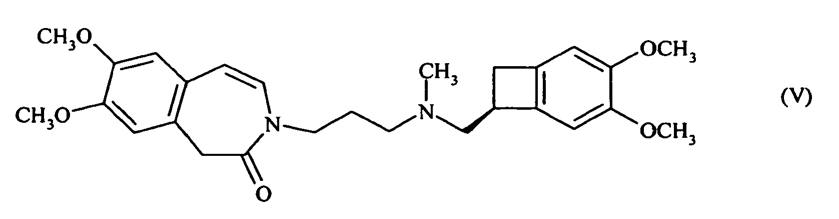 lequel est soumis à une réaction d'hydrogénation pour conduire à l'ivabradine de formule (I), qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

2. Procédé de synthèse selon la revendication 1, **caractérisé en ce que** l'agent de réduction utilisé pour effectuer la réaction d'amination réductrice du composé de formule (VI) par le composé de formule (VII) est choisi parmi le triacétoxyborohydrure de sodium, le cyanoborohydrure de sodium, et le dihydrogène en présence d'un catalyseur tel que le palladium, le platine, le nickel, le ruthénium, le rhodium, ainsi que leurs dérivés, notamment sous forme supportée ou sous forme d'oxydes.

3. Procédé de synthèse selon la revendication 2, **caractérisé en ce que** l'agent de réduction utilisé pour effectuer la réaction d'amination réductrice du composé de formule (VI) par le composé de formule (VII) est le dihydrogène en présence de palladium sur charbon.

4. Procédé de synthèse selon la revendication 3, **caractérisé en ce que** la réaction d'amination réductrice entre le composé de formule (VI) et le composé de formule (VII) est conduite à une pression de dihydrogène comprise entre 0,5 et 1,5 bar.

5. Procédé de synthèse selon l'une quelconque des revendications 1 à 4, **caractérisé en ce que** le solvant utilisé pour effectuer la réaction d'amination réductrice du composé de formule (VI) par le composé de formule (VII) est choisi parmi le tétrahydrofurane, le dichlorométhane, le 1,2-dichloroéthane, les acétates et les alcools, préférentiellement l'éthanol, le méthanol ou l'isopropanol, le toluène ou le xylène.

6. Procédé de synthèse selon la revendication 5, **caractérisé en ce que** le solvant utilisé pour effectuer la réaction d'amination réductrice du composé de formule (VI) par le composé de formule (VII) est constitué par un mélange d'éthanol et d'eau.

7. Procédé de synthèse selon l'une quelconque des revendications 1 à 6, **caractérisé en ce que** la réaction d'amination réductrice entre le composé de formule (VI) et le composé de formule (VII) est conduite à une température comprise entre 0 et 40°C.

8. Procédé de synthèse selon l'une quelconque des revendications 1 à 7, **caractérisé en ce que** le solvant organique utilisé dans la réaction entre les composés de formule (VIII) et (IX) est choisi parmi le toluène, le dichlorométhane, le 2-méthyltétrahydrofurane, le chlorobenzène, le 1,2-dichloroéthane, le chloroforme et le dioxane.

9. Procédé de synthèse selon la revendication 8, **caractérisé en ce que** le solvant organique utilisé dans la réaction entre les composés de formule (VIII) et (IX) est le dichlorométhane.

10. Procédé de synthèse selon l'une quelconque des revendications 1 à 9, **caractérisé en ce que** la réaction entre les composés de formule (VIII) et (IX) est conduite à une température comprise entre 0 et 40°C.

11. Procédé de synthèse selon l'une quelconque des revendications 1 à 10, **caractérisé en ce que** la base utilisée dans la réaction entre les composés de formule (VIII) et (IX) est choisie parmi la pyridine, la 4-diméthylaminopyridine (DMAP) et une amine tertiaire.

12. Procédé de synthèse selon la revendication 11, **caractérisé en ce que** la base utilisée dans la réaction entre les composés de formule (VIII) et (IX) est la triéthylamine.

13. Procédé de synthèse selon l'une quelconque des revendications 1 à 12, **caractérisé en ce que** l'acide utilisé pour effectuer la cyclisation du composé de formule (X) en composé de formule (V) est choisi parmi l'acide sulfurique concentré, l'acide polyphosphorique, l'acide chlorhydrique concentré en solution aqueuse, l'acide chlorhydrique concentré en solution dans l'acide acétique, l'acide bromhydrique concentré en solution dans l'acide acétique et l'acide méthanesulfonique.

14. Procédé de synthèse selon la revendication 13, **caractérisé en ce que** l'acide utilisé pour effectuer la cyclisation du composé de formule (X) en composé de formule (V) est l'acide chlorhydrique concentré en solution dans l'acide acétique.

15. Procédé de synthèse selon l'une quelconque des revendications 1 à 14, **caractérisé en ce que** la cyclisation du composé de formule (X) en composé de formule (V) est conduite à une température comprise entre 0 et 40°C.

16. Composé de formule (VIII) : 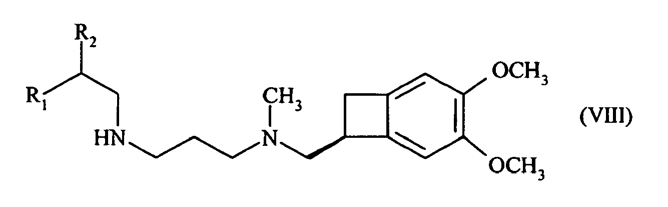 dans laquelle R₁ et R₂, identiques ou différents, représentent des groupements alkoxy (C₁-C₆) linéaires ou ramifiés, ou bien forment ensemble avec l'atome de carbone qui les porte un cycle 1,3-dioxane, 1,3-dioxolane ou 1,3-dioxépane.

17. Composé de formule (X) : 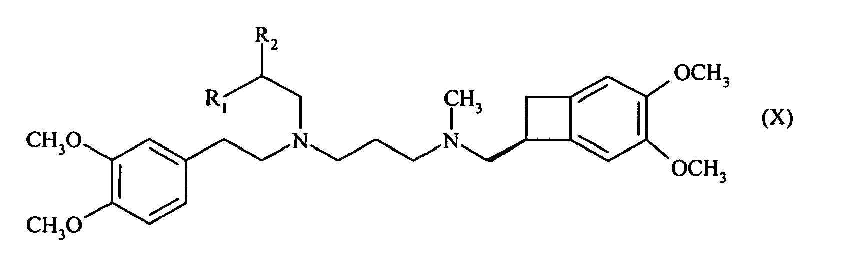 dans laquelle R₁ et R₂ sont tels que définis à la revendication 16.

## Claims

1. Process for the synthesis of ivabradine of formula (I): 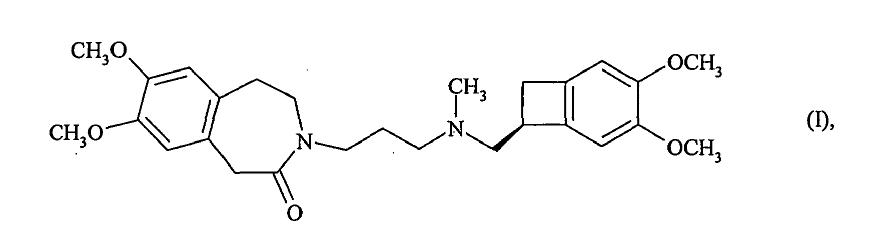 **characterised in that** the compound of formula (VI): 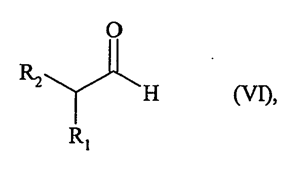 wherein R₁ and R₂, which are the same or different, represent linear or branched (C₁-C₆)alkoxy groups or together with the carbon atom carrying them form a 1,3-dioxane, 1,3-dioxolane or 1,3-dioxepane ring,
is subjected to a reductive amination reaction with the compound of formula (VII): 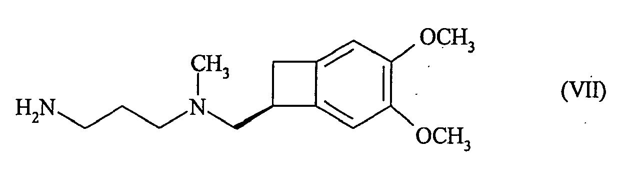 in the presence of a reducing agent,
in an organic solvent, a mixture of organic solvents or a mixture of organic solvent(s) and water,
to yield the compound of formula (VIII): 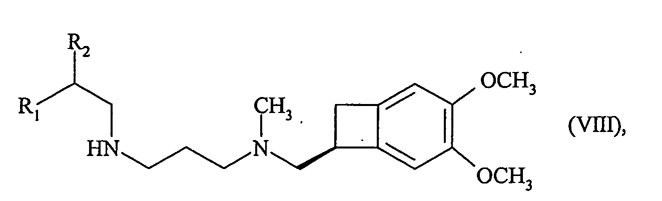 wherein R₁ and R₂ are as defined hereinbefore,
which is subjected to a condensation reaction with the compound of formula (IX): 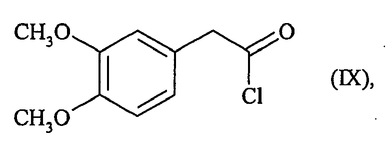 in the presence of a base in an organic solvent, to yield the compound of formula (X): 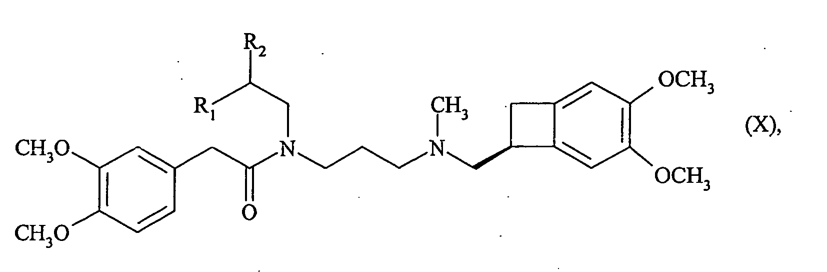 wherein R₁ and R₂ are as defined hereinbefore,
which is subjected to a cyclisation reaction in an acid medium to yield the compound of formula (V): 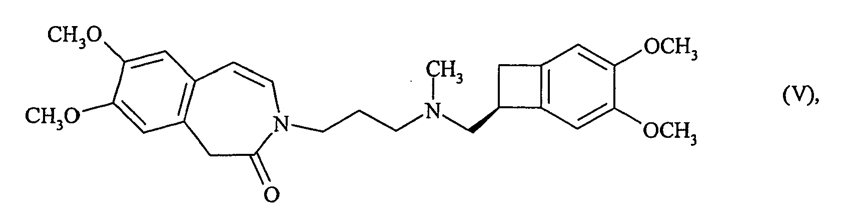 which is subjected to a hydrogenation reaction to yield ivabradine of formula (I), which may optionally be converted into addition salts thereof with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into hydrates thereof.

2. Synthesis process according to claim 1, **characterised in that** the reducing agent used to carry out the reductive amination reaction of the compound of formula (VI) with the compound of formula (VII) is selected from sodium triacetoxyborohydride, sodium cyanoborohydride, and dihydrogen in the presence of a catalyst such as palladium, platinum, nickel, ruthenium, rhodium, and compounds thereof, especially on a support or in the form of oxides.

3. Synthesis process according to claim 2, **characterised in that** the reducing agent used to carry out the reductive amination reaction of the compound of formula (VI) with the compound of formula (VII) is dihydrogen in the presence of palladium-on-carbon.

4. Synthesis process according to claim 3, **characterised in that** the reductive amination reaction between the compound of formula (VI) and the compound of formula (VII) is carried out at a dihydrogen pressure of from 0.5 to 1.5 bar.

5. Synthesis process according to any one of claims 1 to 4, **characterised in that** the solvent used to carry out the reductive amination reaction of the compound of formula (VI) with the compound of formula (VII) is selected from tetrahydrofuran, dichloromethane, 1,2-dichloroethane, acetates and alcohols, preferably ethanol, methanol or isopropanol, toluene and xylene.

6. Synthesis process according to claim 5, **characterised in that** the solvent used to carry out the reductive amination reaction of the compound of formula (VI) with the compound of formula (VII) comprises a mixture of ethanol and water.

7. Synthesis process according to any one of claims 1 to 6, **characterised in that** the reductive amination reaction between the compound of formula (VI) and the compound of formula (VII) is carried out at a temperature of from 0 to 40°C.

8. Synthesis process according to any one of claims 1 to 7, **characterised in that** the organic solvent used in the reaction between the compounds of formulae (VIII) and (IX) is selected from toluene, dichloromethane, 2-methyltetrahydrofuran, chlorobenzene, 1,2-dichloroethane, chloroform and dioxane.

9. Synthesis process according to claim 8, **characterised in that** the organic solvent used in the reaction between the compounds of formulae (VIII) and (IX) is dichloromethane.

10. Synthesis process according to any one of claims 1 to 9, **characterised in that** the reaction between the compounds of formulae (VIII) and (IX) is carried out at a temperature of from 0 to 40°C.

11. Synthesis process according to any one of claims 1 to 10, **characterised in that** the base used in the reaction between the compounds of formulae (VIII) and (IX) is selected from pyridine, 4-dimethylaminopyridine (DMAP) and a tertiary amine.

12. Synthesis process according to claim 11, **characterised in that** the base used in the reaction between the compounds of formulae (VIII) and (IX) is triethylamine.

13. Synthesis process according to any one of claims 1 to 12, **characterised in that** the acid used to carry out cyclisation of the compound of formula (X) to form the compound of formula (V) is selected from concentrated sulphuric acid, polyphosphoric acid, concentrated hydrochloric acid in aqueous solution, concentrated hydrochloric acid in solution in acetic acid, concentrated hydrobromic acid in solution in acetic acid, and methanesulphonic acid.

14. Synthesis process according to claim 13, **characterised in that** the acid used to carry out cyclisation of the compound of formula (X) to form the compound of formula (V) is concentrated hydrochloric acid in solution in acetic acid.

15. Synthesis process according to any one of claims 1 to 14, **characterised in that** cyclisation of the compound of formula (X) to form the compound of formula (V) is carried out at a temperature of from 0 to 40°C.

16. Compound of formula (VIII): 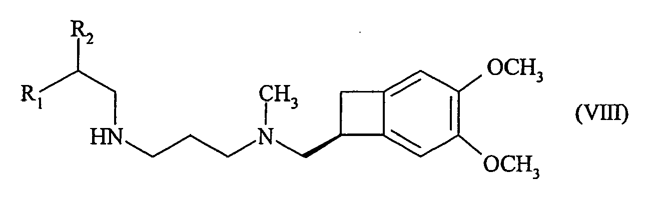 wherein R₁ and R₂, which are the same or different, represent linear or branched (C₁-C₆)alkoxy groups or together with the carbon atom carrying them form a 1,3-dioxane, 1,3-dioxolane or 1,3-dioxepane ring.

17. Compound of formula (X): 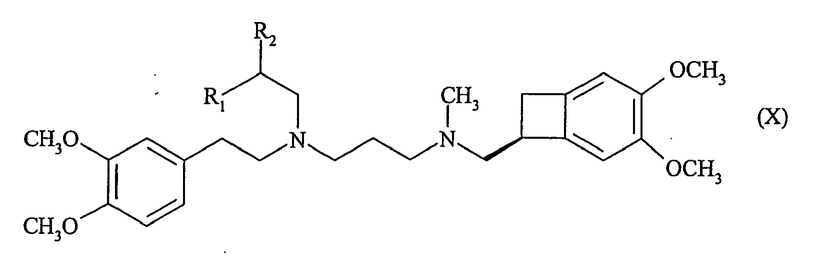 wherein R₁ and R₂ are as defined in claim 16.

## Patentansprüche

1. Verfahren zur Synthese von Ivabradin der Formel (I): 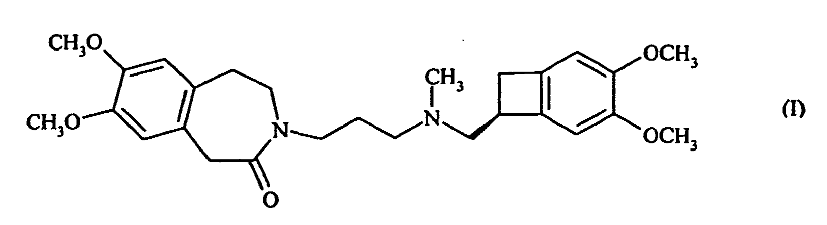 **dadurch gekennzeichnet, dass** man die Verbindung der Formel (VI): 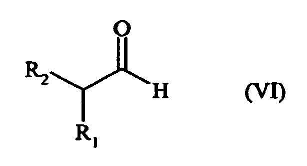 in der R₁ und R₂, die identisch oder verschieden sind, geradkettige oder verzweigte (C₁-C₆)-Alkoxygruppen bedeuten oder gemeinsam mit dem sie tragenden Kohlenstoffatom einen 1,3-Dioxan-, 1,3-Dioxolan- oder 1,3-Dioxepanring bilden,
in Gegenwart eines Reduktionsmittels
in einem organischen Lösungsmittel, einer Mischung aus organischen Lösungsmitteln oder einer Mischung aus einem oder mehreren organischen Lösungsmitteln und Wasser einer reduzierenden Aminierungsreaktion mit der Verbindung der Formel (VII): 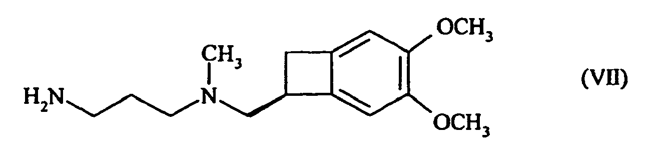 unterwirft zur Bildung der Verbindung der Formel (VIII): 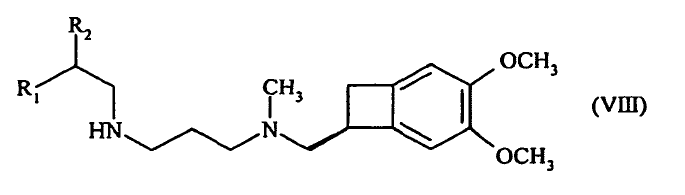 in der R₁ und R₂ die oben angegebenen Bedeutungen besitzen,
welche in Gegenwart einer Base in einem organischen Lösungsmittel einer Kondensationsreaktion mit der Verbindung der Formel (IX): 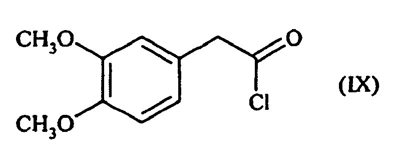 unterworfen wird zur Bildung der Verbindung der Formel (X): 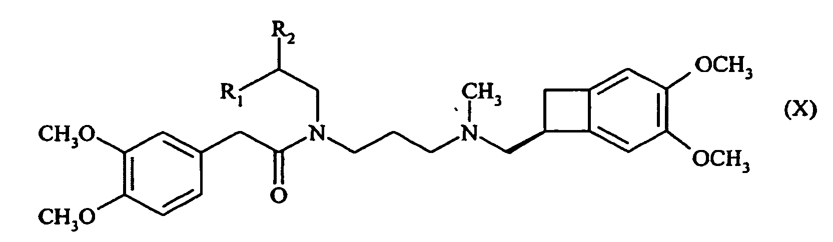 in der R₁ und R₂ die oben angegebenen Bedeutungen besitzen,
welche in saurem Medium einer Cyclisierungsreaktion unterworfen wird zur Bildung der Verbindung der Formel (V): 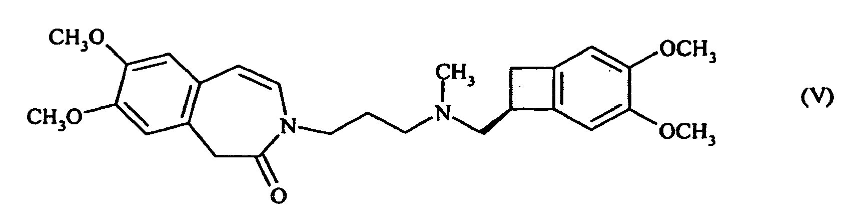 welche einer Hydrierungsreaktion unterworfen wird zur Bildung von Ivabradin der Formel (I), welches gegebenenfalls in eines seiner Additionssalze mit einer pharmazeutisch annehmbaren Säure ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure, und in ihre Hydrate umgewandelt werden kann.

2. Syntheseverfahren nach Anspruch 1, **dadurch gekennzeichnet, dass** das zur Durchführung der reduzierenden Aminierungsreaktion der Verbindung der Formel (VI) mit der Verbindung der Formel (VII) verwendete Reduktionsmittel ausgewählt wird aus Natriumtriacetoxyborhydrid, Natriumcyanborhydrid und Wasserstoff in Gegenwart eines Katalysators, wie Palladium, Platin, Nickel, Ruthenium, Rhodium sowie deren Derivate, insbesondere in auf einem Träger vorliegenden Form oder in Form der Oxide.

3. Syntheseverfahren nach Anspruch 2, **dadurch gekennzeichnet, dass** das zur Durchführung der reduzierenden Aminierungsreaktion der Verbindung der Formel (VI) mit der Verbindung der Formel (VII) verwendete Reduktionsmittel Wasserstoff in Gegenwart von Palladium-auf-Kohlenstoff ist.

4. Syntheseverfahren nach Anspruch 3, **dadurch gekennzeichnet, dass** die reduzierende Aminierungsreaktion zwischen der Verbindung der Formel (VI) und der Verbindung der Formel (VII) bei einem Wasserstoffdruck zwischen 0,5 und 1,5 bar durchgeführt wird.

5. Syntheseverfahren nach einem der Ansprüche 1 bis 4, **dadurch gekennzeichnet, dass** das zur Durchführung der reduzierenden Aminierungsreaktion der Verbindung der Formel (VI) mit der Verbindung der Formel (VII) verwendete Lösungsmittel ausgewählt wird aus Tetrahydrofuran, Dichlormethan, 1,2-Dichlorethan, Acetaten und Alkoholen, vorzugsweise Ethanol, Methanol oder Isopropanol, Toluol oder Xylol.

6. Syntheseverfahren nach Anspruch 5, **dadurch gekennzeichnet, dass** das zur Durchführung der reduzierenden Aminierungsreaktion der Verbindung der Formel (VI) mit der Verbindung der Formel (VII) verwendete Lösungsmittel eine Mischung aus Ethanol und Wasser ist.

7. Syntheseverfahren nach einem der Ansprüche 1 bis 6, **dadurch gekennzeichnet, dass** die reduzierende Aminierungsreaktion zwischen der Verbindung der Formel (VI) und der Verbindung der Formel (VII) bei einer Temperatur zwischen 0 und 40 °C durchgeführt wird.

8. Syntheseverfahren nach einem der Ansprüche 1 bis 7, **dadurch gekennzeichnet, dass** das bei der Reaktion der Verbindungen der Formeln (VIII) und (IX) verwendete organische Lösungsmittel ausgewählt wird aus Toluol, Dichlormethan, 2-Methyltetrahydrofuran, Chlorbenzol, 1,2-Dichlorethan, Chloroform und Dioxan.

9. Syntheseverfahren nach Anspruch 8, **dadurch gekennzeichnet, dass** das bei der Reaktion zwischen den Verbindungen der Formeln (VIII) und (IX) verwendete organische Lösungsmittel Dichlormethan ist.

10. Syntheseverfahren nach einem der Ansprüche 1 bis 9, **dadurch gekennzeichnet, dass** die Reaktion zwischen den Verbindungen der Formeln (VIII) und (IX) bei einer Temperatur zwischen 0 und 40 °C durchgefüht wird.

11. Syntheseverfahren nach einem der Ansprüche 1 bis 10, **dadurch gekennzeichnet, dass** die bei der Reaktion zwischen den Verbindungen der Formeln (VIII) und (IX) verwendete Base ausgewählt wird aus Pyridin, 4-Dimethylaminopyridin (DMAP) und einem tertiären Amin.

12. Syntheseverfahren nach Anspruch 11, **dadurch gekennzeichnet, dass** die bei der Reaktion zwischen den Verbindungen der Formeln (VIII) und (IX) verwendete Base Triethylamin ist.

13. Syntheseverfahren nach einem der Ansprüche 1 bis 12, **dadurch gekennzeichnet, dass** die zur Durchführung der Cyclisierung der Verbindung der Formel (X) zu der Verbindung der Formel (V) verwendete Säure ausgewählt wird aus konzentrierter Schwefelsäure, Polyphosphorsäure, konzentrierter Chlorwasserstoffsäure in wässriger Lösung, konzentrierter Chlorwasserstoffsäure in Lösung in Essigsäure, konzentrierter Bromwasserstoffsäure in Lösung in Essigsäure und Methansulfonsäure.

14. Syntheseverfahren nach Anspruch 13, **dadurch gekennzeichnet, dass** die zur Durchführung der Cyclisierung der Verbindung der Formel (X) zu der Verbindung der Formel (V) verwendete Säure konzentrierte Chlorwasserstoffsäure in Lösung in Essigsäure ist.

15. Syntheseverfahren nach einem der Ansprüche 1 bis 14, **dadurch gekennzeichnet, dass** die Cyclisierung der Verbindung der Formel (X) zu der Verbindung der Formel (V) bei einer Temperatur zwischen 0 und 40 °C durchgeführt wird.

16. Verbindung der Formel (VIII): 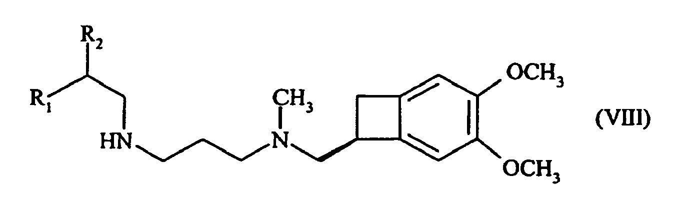 in der R₁ und R₂, die identisch oder verschieden sind, geradkettige oder verzweigte (C₁-C₆)-Alkoxygruppen bedeuten oder gemeinsam mit dem sie tragenden Kohlenstoffatom einen 1,3-Dioxan-, 1,3-Dioxolan- oder 1,3-Dioxepanring bilden.

17. Verbindung der Formel (X): 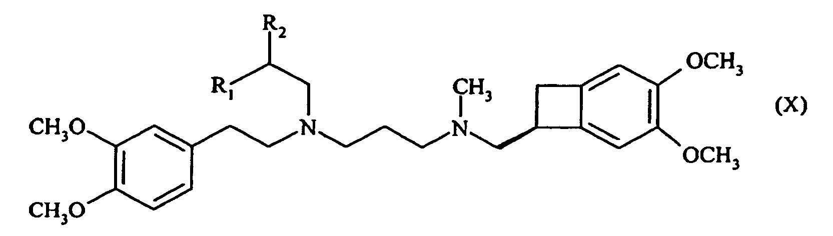 in der R₁ und R₂ die in Anspruch 16 angegebenen Bedeutungen besitzen.
